# EUROPEAN PATENT APPLICATION

(11) **EP 4 342 410 A1**
(43) Date of publication of application: **27.03.2024**
(21) Application number: 23150532.2
(22) Date of filing: 06.01.2023
(51) Int. Cl.: A61B 34/10, A61B 17/00, A61B 90/00

(54) **RE-ROUTING IN LUNG-RELATED INTERVENTIONS**

(30) Priority: 22.09.2022 US 202263408873 P
(71) Applicant: Koninklijke Philips N.V., 5656 AG Eindhoven (NL)
(72) Inventor: LEE, Brian C, Eindhoven (NL); SINHA, Ayushi, Eindhoven (NL); VARBLE, Nicole, 5656AG Eindhoven (NL)
(74) Representative: Philips Intellectual Property & Standards

(57) **Abstract**

The present invention relates to routing in lung-related interventions. In order to provide further improved guidance in lung-related interventions, a device (10) for re-routing a planned pathway for an interventional device in lung-related interventions is provided. The device comprises a data input (12), a data storage (14), a data processor (16) and an output interface (18). The data input is configured to provide a current location of the interventional device in relation to the pre-operative 3D model. The data storage is configured to provide a pre-operative 3D model of at least a part of the airways of a subject currently undergoing a lung-related procedure, and to provide at least one 3D model of at least one available interventional device and physical parameters associated with the at least one available interventional device. The data processor is configured to calculate a plurality of pathway candidates from the current location to the target location taking into account the at least one 3D model and the associated physical parameters of the at least one available interventional device upon receiving a triggering signal for re-routing during a lung-related navigation procedure that comprises a pre-procedurally planned pathway to a target location. For the pathway candidates' calculation, the data processor is configured to simulate a plurality of pathway candidates. For the simulating, the data processor is configured to determine device-pathway compatibility of the simulated pathway candidates, and to evaluate the simulated pathway candidates for selection of at least one candidate. The output interface is configured to provide at least one simulated pathway candidate for further navigation of the interventional device.

## Description

### FIELD OF THE INVENTION

The present invention relates to routing in lung-related interventions. The invention relates in particular to a device for re-routing a planned pathway for an interventional device in lung-related interventions, to a system for lung-related navigation guidance and to a method for re-routing a planned pathway for an interventional device in lung-related interventions.

### BACKGROUND OF THE INVENTION

As an example relating to lung bronchoscopy, peripheral lung nodules are widely considered to be challenging trans-bronchial biopsy (TBB) and transbronchial needle aspiration (TBNA) targets due to the difficulty of navigating catheters and biopsy devices through the narrow and complex peripheral airways. One example for navigation support are CT-guided percutaneous procedures. For example, they might show high diagnostic yield, e.g. showing approximately 90% diagnostic yield. Another example are trans-bronchial methods that have a larger safety factor for the patient particularly in reducing the risk of dangerous complications like pneumothorax and haemorrhage. One of the main roadblocks for increasing diagnostic yield in peripheral TBNA may be the challenging navigational setting. However, it has been shown that although modern navigational technologies such as endobronchial ultrasound (EBUS), radial endobronchial ultrasound (r-EBUS), and electromagnetic navigation (EMN) have made transbronchial biopsy and needle aspiration a more attractive proposition, their successful usage remains low in peripheral lesions relative to other targets and trans-thoracic methods.

### SUMMARY OF THE INVENTION

There may thus be a need to provide further improved guidance in lung-related interventions.

The object of the present invention is solved by the subject-matter of the independent claims; further embodiments are incorporated in the dependent claims. It should be noted that the following described aspects of the invention apply also for the device for re-routing a planned pathway for an interventional device in lung-related interventions, for the system for lung-related navigation guidance and for the method for re-routing a planned pathway for an interventional device in lung-related interventions.

According to the present invention, a device for re-routing a planned pathway for an interventional device in lung-related interventions is provided. The device comprises a data input, a data storage, a data processor and an output interface. The data input is configured to provide a current location of the interventional device in relation to the pre-operative 3D model. The data storage is configured to provide a pre-operative 3D model of at least a part of the airways of a subject currently undergoing a lung-related procedure. The data storage is also configured to provide at least one 3D model of at least one available interventional device and physical parameters associated with the at least one available interventional device. The data processor is configured to calculate a plurality of pathway candidates from the current location to the target location taking into account the at least one 3D model and the associated physical parameters of the at least one available interventional device upon receiving a triggering signal for re-routing during a lung-related navigation procedure that comprises a pre-procedurally planned pathway to a target location. For the pathway candidates' calculation, the data processor is configured to simulate a plurality of pathway candidates. Further, for the simulating, the data processor is configured to determine device-pathway compatibility of the simulated pathway candidates, and to evaluate the simulated pathway candidates for selection of at least one candidate. The output interface is configured to provide at least one simulated pathway candidate for further navigation of the interventional device.

As an effect, accessibility of a peripheral lesion by a certain device being steered through the peripheral airways is determined.

To navigate to an approach or target, the device geometry is considered. The compatibility of different guiding sheathes for the purpose of lung bronchoscopy and their limitations in terms of the delivery angle of both the sheath and the needle are simulated.

According to an example, the physical parameters comprise at least one of the group of: flexibility of the device, variations of the flexibility along the length of the device, bending radius of the device, variations of the bending radius along the length of the device, surface smoothness and resulting friction with adjacent or abutting tissue surfaces and size of the device.

This provides the advantage that the routing to the target can be better adapted to the respective device used for maneuvering through the airways structure.

According to an example, for the simulating, at least one parameter of the group of: type of the interventional device, flexibility of the interventional device, radius of the interventional device, imaging device type, device tip size, needle or forceps size and navigational pathway, is varied.

By comparing e.g. examples of two different needle delivery devices with varying delivery angles as well as with several sheath/needle combinations, an optimized re-routing is provided.

Varying the delivery angle also improves the success rate of reaching the target biopsy site depending on the lesion size, the needle size, and the type of guiding sheath, indicating that the selection of pathway and deployed devices constrain one another.

According to an example, the data input is configured to receive a user interaction for identifying a location of a blockage in the airways of the subject. The data processor is configured to consider the blockage when simulating the pathway candidates.

As an advantage, the user can add navigation-relevant information which is used for the routing computation.

According to an example, possible simulated pathway candidates are provided to the user for user selection.

The user can this select an appropriate route.

According to an example, for the evaluating, at least one of the following criteria is measured: amount of target tissue able to be biopsied, avoidance of critical surroundings comprising at least one of the group of critical vasculature and critical nerve tracts, pathway navigability and fluoroscopy view quality.

This allows to identify the suitability of a pathway under a respective aspect.

According to an example, the data processor is configured: to numerically assess the measured criteria; to select the simulated pathway with the highest scoring; and to suggest the pathway as a re-routed pathway for an updated navigation to the target location.

Such an assessment provides an attempt to provide the user with an objective selection process, thus further facilitating the user's workflow.

According to an example, a plurality of 3D models of a set of available devices in the operating room is provided. For the simulating, the data processor is configured to base the variation of at least one parameter upon the set of the available devices.

This allows to identify the usability of other available devices, which further improves the navigation procedure.

According to an example, the data processor is configured to generate a simulated fluoroscopy view of the target site for a given approach angle of the respective simulated pathway and to present same to the user.

The simulated view provides a source of information familiar to the user due to the similar appearance with a fluoroscopy image.

According to the present invention, a system for lung-related navigation guidance is provided. The system comprises a device for re-routing a planned pathway for an interventional device in lung-related interventions according to one of the preceding examples. The system also comprises an interventional device position determining arrangement. The system further comprises a user interaction interface and a display arrangement. The user interaction interface is configured for generating a triggering signal for re-routing during a lung-related navigation procedure. The interventional device position determining arrangement is configured to determine a current location of the interventional device upon the triggering signal is generated. The device for re-routing a planned pathway provides at least one simulated pathway acting as navigation for pursuing with moving the interventional device towards the target; and wherein the display arrangement is configured to provide the at least one simulated pathway.

According to the present invention, a method for re-routing a planned pathway for an interventional device in lung-related interventions is provided. The method comprises the following steps: - providing a pre-operative 3D model of at least a part of the airways of a subject currently undergoing the lung-related procedure; and providing at least one 3D model of at least one available interventional device and physical parameters associated with the at least one available interventional device; upon receiving a triggering signal for re-routing during a lung-related navigation procedure that comprises a pre-procedurally planned pathway to a target location: - providing a current location of the interventional device in relation to the pre-operative 3D model; - calculating a plurality of pathway candidates from the current location to the target location taking into account the at least one 3D model and the associated physical parameters of the at least one available interventional device. For the pathway candidates' calculation, it is provided simulating a plurality of pathway candidates. Further, the simulating comprises the steps of: determining device-pathway compatibility of the simulated pathway candidates; and evaluating the simulated pathway candidates for selection of a best candidate.

The combination of devices may also constrain each other. For instance, a guiding sheath placed optimally near a lesion to be biopsied may be straightened out once a needle is inserted through it, potentially making the positioning of the combination of devices non-optimal for the biopsy. Each factor additionally may have trade-offs; for instance, reducing the needle size improves steerability and increases the range of successful delivery angles but reduces the size of the biopsied tissue sample as well as its reach outside the airways for transbronchial biopsy.

In addition to the device selection and pathway tortuosity, patient safety is also of concern. Proximity of the navigation pathway or the needle deployment site to major blood vessels or other critical structures is considered to avoid negative outcomes.

As an example, the present invention is intended for peripheral lung bronchoscopy pathway planning and re-routing, however similar methods can also be applied to vascular navigation and re-routing, as well as navigation of any endoscopic device through an anatomy where several pathways are possible.

The present invention provides diversions in a time-saving manner and increases the likelihood that the task of the user is ultimately successful. In view of the local geometry of the airways relative to the biopsy device size and shape, also addressing imaging conditions for that approach angle, the result of sampling of tissue away from the preferred target location or failure to biopsy is avoided and thus prevented altogether. Particularly in the case of trans-bronchial lesions, i.e. lesions that exist outside of the airways themselves, several pathways may exist to reach the same target location, with variations in which distal branch to access and where to puncture the airway wall in order to reach the target location. The accessibility of each option may not be apparent until the time of the procedure. The present invention enables to optimize the routing procedure during the actual device movement by the user.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for re-routing a planned pathway for an interventional device in lung-related interventions.
Fig. 2 shows an example of a system for lung-related navigation guidance.
Fig. 3a and Fig. 3b show an example in which sample pathway/device selection is shown to the user after simulation.
Fig. 4 shows basic steps of an example of a method for re-routing a planned pathway for an interventional device in lung-related interventions.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

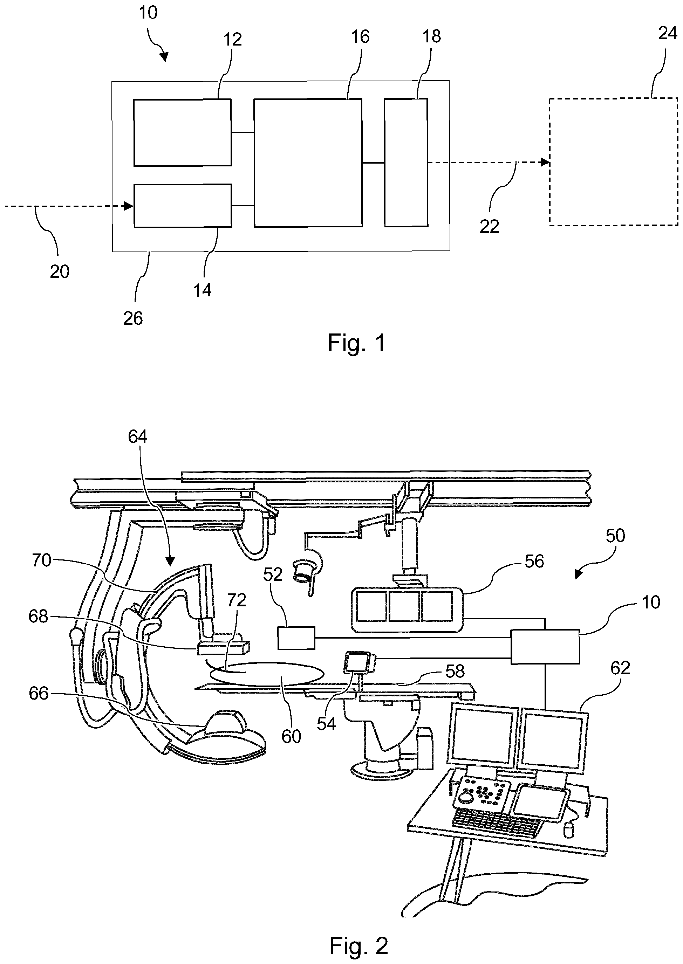

Fig. 1 schematically shows an example of a device 10 for re-routing a planned pathway for an interventional device in lung-related interventions. The device 10 comprises a data input 12, a data storage 14, a data processor 16 and an output interface 18. The data input 12 is configured to provide a current location of the interventional device in relation to the pre-operative 3D model. The data storage 14 is configured to provide a pre-operative 3D model of at least a part of the airways of a subject currently undergoing a lung-related procedure. The data storage 14 is also configured to provide at least one 3D model of at least one available interventional device and physical parameters associated with the at least one available interventional device. The data processor 16 is configured to calculate a plurality of pathway candidates from the current location to the target location taking into account the at least one 3D model and the associated physical parameters of the at least one available interventional device upon receiving a triggering signal for re-routing during a lung-related navigation procedure that comprises a pre-procedurally planned pathway to a target location. For the pathway candidates' calculation, the data processor 16 is configured to simulate a plurality of pathway candidates. Further, for the simulating, the data processor 16 is configured to determine device-pathway compatibility of the simulated pathway candidates, and to evaluate the simulated pathway candidates for selection of at least one candidate. The output interface 18 is configured to provide at least one simulated pathway candidate for further navigation of the interventional device.

A first arrow 20 in broken lines indicates the data input of e.g. the current location of the interventional device in relation to the pre-operative 3D model.

A second arrow 22 in broken lines indicates the data output of e.g. the at least one simulated pathway candidate for further navigation of the interventional device.

A broken frame 24 in broken lines indicates a display as an example for presenting the simulated pathway candidate to a user.

A frame 26 indicates an option according to which the data input 12, the data storage 14, the data processor 16 and the output interface 18 are provided in an integrated manner, such as in a common housing. However, they can also be arranged integrated in different combinations or even as separate components.

In an example, the pre-operative 3D model comprises of at least a part of a tree structure of the airways of the subject.

The plurality of pathway candidates provide multiple routes from which one is then selected for the navigation or steering of the interventional device moved at least partly within an airway structure of the subject.

Using a simulation, it is enabled to determine whether the current selected pathway is compatible for the current selected device, and also to evaluate the quality of the selection in terms of the parameters described above. The simulation comprises two steps: 1) determining device/pathway compatibility and 2) evaluating pathway quality.

The simulation is triggered by the physician during a surgical procedure, when it is determined that the planned pathway has failed to reach the target site and a new pathway is required.

In the first step, the simulation determines for a set of available device(s) in the operating room whether or not their geometries are physically capable of reaching the target site via the selected pathway. As an example, different ways to get this information are provided, e.g. system file, device recognition and the like.

In an example, a device compatibility simulation with high complexity includes physics-based modelling of the device passing through a number of candidate pathways, using a patient-specific airway model derived from pre-operative imaging. A potential advantage can be provided if the used manufactured devices have physical properties that are known and CAD models are readily available and integrated into an operating and imaging system.

As an example, the factors involved in the pathway selection process include:
- device parameters, e.g. needle/catheter/imager(s) size, shape, flexibility;
- pathway navigability, e.g. airway diameter, bending angle, tortuosity;
- device navigability at the distal end, e.g., although the flexibility of the device may be known, the user's ability to affect the distal end of the device by manipulating the proximal end may be limited based on its current configuration and anatomical location, hence the need for simulation;
- biopsy site, e.g. needle approach angle, biopsy sample size/location, overlap with target lesion;
- safety factors, e.g. proximity to vessels/structures; and
- viewing angle, e.g. ability to see the target and its relation to the device on its approach in the intraoperative modality from the given configuration.

In an example, a model makes stepwise comparisons between the device and pathway. The inputs to a simpler simulation could include: 1. device parameters Dᵢ = {d*_{θ}*, dᵣ, ... } where d*_{θ}* indicates the bending limits of each segment of the device and dᵣ indicates the radius of the device along its length, etc. Here, the "device" includes the guiding catheter, imaging device(s), and biopsy device for each combination of devices **I**; and 2. airway parameters Aⱼ = {a*_{θ}*, aᵣ,... } where a*_{θ}* indicates the minimum bending angle required to pass each segment of the airway, aᵣ indicates the radius of the airway over the length of the path, etc. for a set of candidate pathways **j**. Given these inputs, the simulator determines whether any physical constraints of the airways exceed the limits of the combined device, for each set of devices and airways.

The present invention addresses the dependence of some of the above factors on others that creates a high dimensional problem that is solved on-the-fly by the simulation provided as intraoperative re-routing which considers the possibility of optimizing for multiple device options. While pre-procedural planning is based on a pre-procedural knowledge, the re-routing of the present invention allows to consider knowledge acquired during the operation.

In an example, a physics based modelling is provided that is based on devices whose properties are known in advance.

The term "data input" relates to providing or supplying data for data processing steps. The data input can also be referred to as image data input. The data input can also be referred to as data supply, as image data supply, as image input, as input unit or simply as input. In an example, the image data input is data-connectable to an imaging source arrangement.

The term "data processor" relates to a processor or part of a processor arrangement that is provided to conduct the computing steps using the data supplied by the data input. The data processor can also be referred to as data processing arrangement, as processor unit or as processor. In an example, the data processor is data-connected to the data input and the output interface.

The term "output interface" relates to an interface for providing the processed or computed data for further purposes. The output interface can also be referred to as output or output unit. In an example, the output interface is data-connectable to a display arrangement or display device. In another example, the output is data-connected to a display. As an example, the signals by the controller can be provided by the output interface.

The term "current location" relates to a position of the device achieved during an actual guiding movement. As an example, the current location refers to the front (or distal) tip of the interventional device.

In an example, the interventional device is provided as a navigating catheter, or as a biopsy device or as an imaging device.

The term "pathway candidates" relates to pathways, i.e. routes, identified by the data processor which are suitable for the navigation towards the target. The pathway candidates may be suitable for the currently provided interventional device. In an option, the pathway candidates also relate to pathways suitable for one or more other possibly available interventional devices.

The term "device-pathway compatibility" relates to the identified pathway, i.e. the general possible route, and its capability for being suitable for a certain device. It is checked or verified that a certain interventional device can actually maneuver through the identified pathway.

The term "evaluate" relates to determining or analyzing the suitability of a pathway compared to other pathways. The evaluation can also be referred to as assessment or weighting based on the values for the respective pathways. The evaluation is provided in order to identify the most promising pathways, or even the best pathway. The evaluation may also comprise different value categories and may provide the values to the user. For example, a certain pathway may be shorter than other pathways, but it may be more difficult to steer the device along that pathway. Another example is a pathway that is less complex, but which results in a suboptimal angle for performing the biopsy action

Examples for lung interventions are lung biopsy procedures and lung bronchoscopy procedures.

In an example, not further shown in detail in Fig. 1, the physical parameters comprise at least one of the group of: flexibility of the device, variations of the flexibility along the length of the device, bending radius of the device, variations of the bending radius along the length of the device, surface smoothness and resulting friction with adjacent or abutting tissue surfaces and size of the device.

In an example, not further shown in detail in Fig. 1, for the simulating, at least one parameter of the group of: type of the interventional device, flexibility of the interventional device, radius of the interventional device, imaging device type, device tip size, needle or forceps size and navigational pathway, is varied.

In an example, not further shown in detail in Fig. 1, the data input 12 is configured to receive a user interaction for identifying a location of a blockage in the airways of the subject. The data processor 16 is configured to consider the blockage when simulating the pathway candidates.

This allows the user to modify the information which is the basis for further route determination. In an option, the blockage is transferred or entered into the 3D model of the airway structure.

In an example, not further shown in detail in Fig. 1, possible simulated pathway candidates are provided to the user for user selection.

Finally, a set of top device/pathway candidates is shared with the physician for final review. As an option, warnings or other signals are indicated if a device change is required for a particular pathway, for instance, a smaller device that would have a higher chance of reaching the target, or if the currently deployed device is incompatible with all candidate pathways or if the quality score of all pathways with the current device is below a threshold. The simulated fluoroscopy view of the target site for the given approach angle is also shown.

In an example, not further shown in detail in Fig. 1, for the evaluating, at least one of the following criteria is measured: amount of target tissue able to be biopsied, avoidance of critical surroundings comprising at least one of the group of critical vasculature and critical nerve tracts, pathway navigability and fluoroscopy view quality.

The term "critical" relates to vessels or nerve tracts which might lead to severe injury or complications when affected during a biopsy or other interventional procedure.

In a second step, compatible pathways are evaluated for quality and the best options are presented to the user. In an example, quality is determined by measuring:
1. The amount of target tissue able to be biopsied, based on the needle approach angle and the needle size: This can be computed by measuring the overlap between the simulated needle's position when fully deployed and the segmented lesion from pre-operative imagery, once the device has reached the target site. The quality can be computed as a function of the tissue volume, or a binary value indicating whether or not some threshold volume has been reached.
2. The ability to avoid critical vasculature: The quality can be computed by measuring the minimum distance of the needle from any major blood vessels throughout the simulated procedure.
3. The pathway navigability: The quality of pathway navigability can be measured by the maximum bending angle or the maximum strain placed on the device during operation as computed by the above simulation. Pathways that are less tortuous will be easier to traverse.
4. The fluoroscopy view quality: The quality of the fluoroscopy view at the target site is valuable for the physician to confirm that a biopsy has been successful. This can be estimated by computing digitally reconstructed radiographs through a pre-operative computed tomography volume and varying the c-arm position until the view with the least obstruction is found (obstructions may include bony structures, etc.). Additionally, a view which is coplanar with the direction of device movement towards the target is optimal for assessing device penetration into the target. The degree of obstruction and device visibility for each pathway's approach angle serves as a measurement of the view quality.

In an example, not further shown in detail in Fig. 1, the data processor 16 is configured to numerically assess the measured criteria. The data processor 16 is also configured to select the simulated pathway with the highest scoring. The data processor 16 is further configured to suggest the pathway as a re-routed pathway for an updated navigation to the target location.

In an example, for the providing of the pre-operative 3D model of at least a part of the tree structure, the data processor 16 is configured to provide a pre-operative 3D model of at least a part of a lung of a subject currently undergoing the lung-related procedure; to segment the airways from the pre-operative 3D model. The data processor 16 is also configured to extract the tree structure of the airways.

In an example, a pre-operative 3D image is provided instead of the pre-operative 3D model of the lung.

In an example, not further shown in detail in Fig. 1, a plurality of 3D models of a set of available devices in the operating room is provided. For the simulating, the data processor 16 is configured to base the variation of at least one parameter upon the set of the available devices.

In other words, for the variation of the parameters, only those parameter combinations are applied, which are actually available in the operating room.

In an option, a further plurality of 3D models of a set of known devices is provided that are currently not available, but which could be arranged within a preset amount of time.

In an example, not further shown in detail in Fig. 1, the data processor 16 is configured to provide a change-indicator to the user if a simulated pathway is determined that is based upon one of the available, but not currently used devices. The change-indicator informs the user that a device change is required for continuing in navigating the device towards the target along the simulated pathway.

In an example, the data processor 16 is configured to determine a probability index for a simulated pathway and to provide the probability index to the user. This may be useful, for example, in cases where the information about the actual airway structure and geometry is not complete.

In an example, not further shown in detail in Fig. 1, the data processor 16 is configured to generate a simulated fluoroscopy view of the target site for a given approach angle of the respective simulated pathway and to present same to the user.

In an example, not further shown in detail in Fig. 1, for the evaluating, the data processor 16 is configured to take a user's prior experience and expertise into account for recommending a pathway and device combination.

In an example, a workflow and setup is provided that comprises:
- a pre-operative 3D model of the individual patient's lungs or anatomy to be operated on; this model could take the form of a point cloud, mesh, volumetric image (for instance, computed tomography), or otherwise;
- a set of 3D models of the available interventional devices that may be used during the procedure; these devices may include but are not limited to catheters, guiding sheathes, biopsy devices (needles, forceps, etc.), imaging devices (bronchoscopes, EBUS/r-EBUS probes, etc.); additionally, the physical parameters associated with each device, such as the radius of the device as a function of its length, the maximum bend angle over its length, the maximum strain the device can undergo, etc.;
- a pre-operative image processing module segments the airways from the pre-operative image or model, and extracts the tree structure of the airways (using the centerlines or machine learning or other method);
- a simulation module that initiates a simulation to determine device/pathway compatibility; the simulation varies all controllable parameters among the possible devices and pathways to determine which are physically feasible; this may comprise of a physics-based simulation using the 3D models of the above second element and the airway segmentation of the above third element, which tests the traversal of the device through the airway and verifies whether or not any impassable collisions or turns may exist;
- a simulation module that evaluates all candidate device/pathway combinations from the above fourth element and produces a measure that describes the quality of each option.

In another embodiment, a simulation is provided that compares the maximum bend angle and other constraints of the devices with the required bend angle and other constraints of the airways for any discrepancies.

In another embodiment, a neural network may be trained to evaluate compatibility between device types and segmented airways.

The parameters to be varied may include: catheter type/flexibility/radius; imaging device type and combinations; needle/forceps size; and navigational pathway.

The measurement is derived from:
- biopsy quality: the amount of tissue overlap between the fully deployed needle and the target lesion, as determined by the position of the device relative to the target lesion in the simulation of the above fourth element;
- safety measurement: the distance of the needle from any critical anatomical structures throughout the procedure;
- pathway navigability measurement: the "difficulty" of traversing each of the selected pathways, estimated by the amount of tortuosity of the pathway or the maximum required bending angle or other parameter; and
- view quality: the amount of occlusion due to anatomical structures of the C-arm viewing angles associated with the particular needle approach angle as determined by the simulation of the above fourth element. The C-arm viewing angle for each pathway/device combination may be the minimally occluded view which is orthogonal to the needle travel direction that contains the needle and the lesion. The view may be simulated from a pre-operative CT image by producing a DRR.
- a processing controller that weights the quality measurements of the above fifth element and selects a set of the best candidate pathway/device combinations for the user.
- a visualization module that displays the candidate pathway/device combinations along with the quality measures and the simulated fluoroscopy image from the candidate view angle and alerts the user if a higher quality option exists that would require the deployed device to be changed, and allows the user to decide whether or not the time spent changing the device is worth using an easier pathway.

As an option, a user interface element is provided that initiates the simulation and visualization procedure of the above fourth to seventh elements.

In an example, it is additionally taking into account the user's prior experience and expertise to make personalized recommendations. Certain paths and maneuvers that may be routine for experienced users, may still present challenges for novice users. Additionally, even some experienced users may not be trained to use certain devices, e.g., R-EBUS. In an example, the system keeps track of the user's ability to successfully navigate to and biopsy lesions using the recommended pathway/device combinations over several procedures and/or over time, and adaptively learns to weight the quality measures such that combinations that the user typically perform well with receive higher weight than combinations that the user finds challenging.

According to an aspect, it is provided to dynamically compute the pathway/device combination that optimizes the navigability of the pathway, the approach angle of the biopsy device, the viewing angle in intraoperative imaging, and patient safety. It is provided to be able to perform this calculation intraoperatively, in order to address the need for fast and accurate re-routing and assessment of the need to switch devices in case that the original planned pathway(s) fail to reach the target site.

According to an aspect, a device selection is added into the framework. This is in particular of advantage when knowledge of catheters/needles are provided that allows to more accurately model compatible device/pathway combinations.

As an effect, bronchoscopy or lung biopsy procedure times are reduced in cases where a deviation from the original planned path must be made.

As an option, automatic and dynamic pathway re-routing is provided in the event that planned pathways fail.

As another option, device selection/change recommendation is provided in the event that the currently deployed device is incompatible with a newly computed path.

As a further option, fluoroscopic view recommendation is provided for a new pathway-anatomy configuration.

Fig. 2 shows an example of a system 50 for lung-related navigation guidance. The system 50 comprises an example of the device 10 for re-routing a planned pathway for an interventional device in lung-related interventions according to one of the preceding examples. Further, an interventional device position determining arrangement 52 is provided. Further, the system 50 comprises a user interaction interface 54 and a display arrangement 56. The user interaction interface 54 is configured for generating a triggering signal for re-routing during a lung-related navigation procedure. The interventional device position determining arrangement 52 is configured to determine a current location of the interventional device upon the triggering signal is generated. The device 10 for re-routing a planned pathway provides at least one simulated pathway acting as navigation for pursuing with moving the interventional device towards the target. The display arrangement 56 is configured to provide the at least one simulated pathway.

It is noted that the connecting lines between e.g. the interventional device position determining arrangement 52 and the device 10 for re-routing a planned pathway for an interventional device in lung-related interventions, or the display arrangement 56 and the device 10 for re-routing a planned pathway for an interventional device in lung-related interventions, or the device 10 for re-routing a planned pathway for an interventional device in lung-related interventions and the console 62 indicate a data-connection, which can be wire-bound or wireless.

In an example, the interventional device position determining arrangement comprises at least one of the group of an X-ray imaging device, electromagnetic sensors and an ultrasound imaging device.

In Fig. 2, the system 50 for lung-related navigation guidance is shown in the context of an interventional room in a medical facility, for example a cathlab. As an option, a subject support 58 is shown on which a subject 60, e.g. a patient, can be arranged. As an option, a console 62 is shown in the right foreground. Further, as an option, an imaging arrangement 62 is shown. As an example, the imaging arrangement 64 comprises an X-ray imaging device with an X-ray source 66 and an X-ray detector 68 mounted to opposing ends of a movable C-arm 70.

In an example, indicated in Fig. 2 as an option, an interventional device 72 is provided for moving along inside airway structures towards a target.

In an example, a system is provided for optimizing the navigational pathway and the device selection based on the current state of the procedure, in order to maximize the navigability of the pathway, the suitability of the needle approach angle, visibility of the target site on imaging, and avoidance of important vasculature.

The term "subject" may also be referred to as individual. The "subject" may further also be referred to as patient, although it is noted that this term does not indicate whether any illness or disease is actually present with the subject.

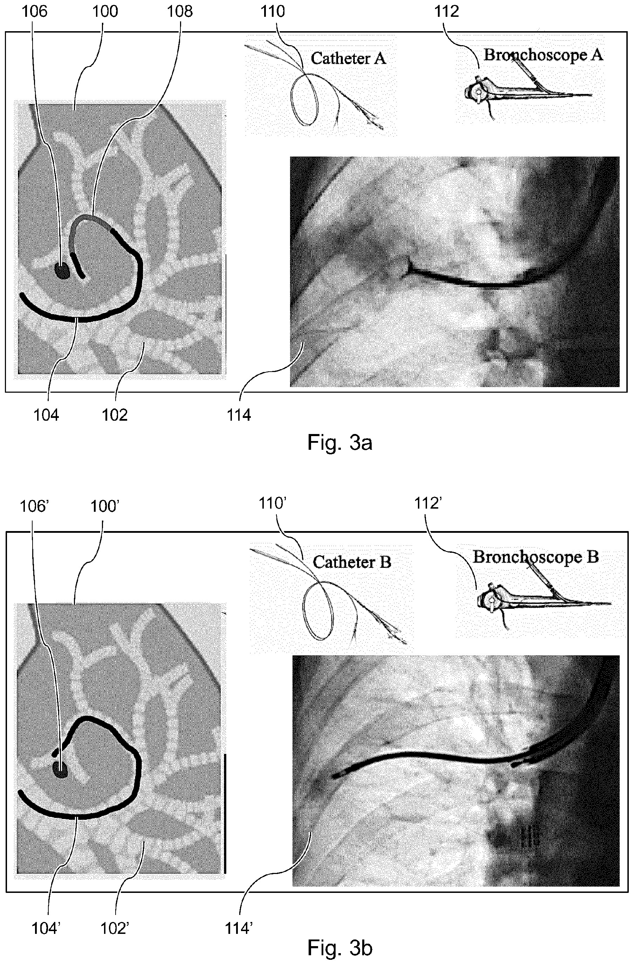

Fig. 3a and Fig. 3b show an example in which sample pathway/device selection is shown to the user after simulation. In Fig. 3a and Fig. 3b, similar elements are indicated with same reference numeral, wherein the index ' is used in Fig. 3b.

A simulation image 100 is shown with a complex airway structure 102. A device 104 is shown inserted along a path towards a target 106. The target lesion site is shown highlighted. An illustration and indication of a certain catheter 110 is shown in the center part. In Fig. 3a, the catheter 110 is labelled as "Catheter A"; in Fig. 3b, the catheter 110' is labelled as "Catheter B". Further, an illustration and indication of a certain bronchoscope 112 is shown. In Fig. 3a, the bronchoscope is labelled as "Bronchoscope A"; in Fig. 3b, the bronchoscope 112' is labelled as "Bronchoscope B".

Further, an optimal digitally reconstructed radiography (DRR) is shown on the bottom right of each option. In Fig. 3a, the digitally reconstructed radiography is indicated with reference numeral 114; in Fig. 3b, the digitally reconstructed radiography is indicated with reference numeral 114'.

As a first option, shown in Fig. 3a, a device combination of catheter A with bronchoscope A is provided, and a potentially high bend angle area highlighted. As a second option, a more favorable route is provided in terms of navigation but the simulated DRR of the approach has a more obscured view of the lesion and device tip.

As an effect, support is provided for the challenging navigational problem posed by the small diameter and tortuosity of the peripheral airways, e.g. when a bronchoscopist encounters the need to divert from the original pathway that was planned prior to the procedure when attempting to navigate to peripheral lesions.

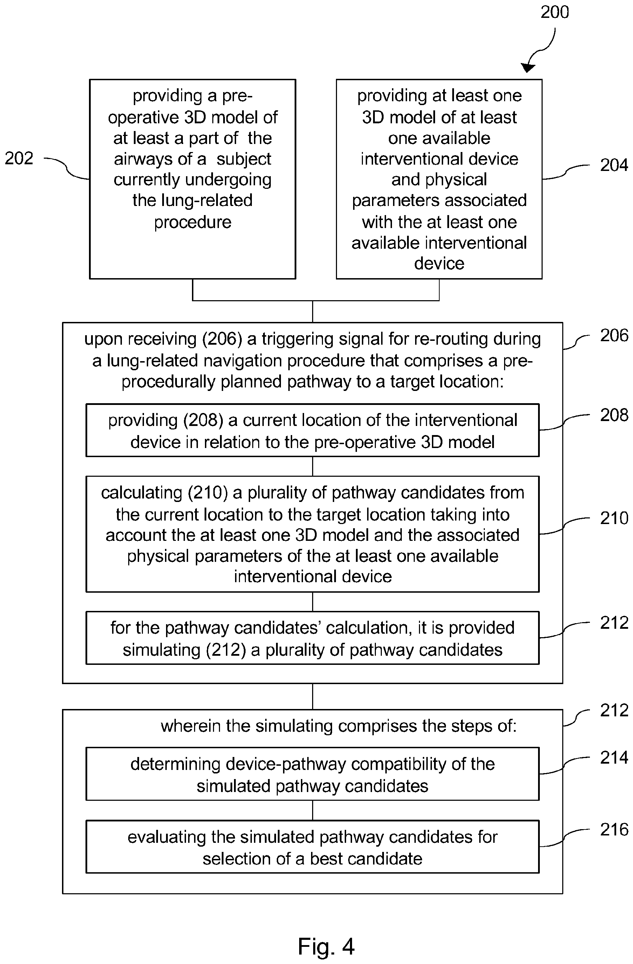

Fig. 4 shows basic steps of an example of a method 200 for re-routing a planned pathway for an interventional device in lung-related interventions. The method 200 comprises the following steps: As a first sub-step 202 of a first step, a pre-operative 3D model of at least a part of the airways of a subject currently undergoing the lung-related procedure is provided;. As a second sub-step 204 of the first step, at least one 3D model of at least one available interventional device and physical parameters associated with the at least one available interventional device is provided. Upon a second step 206, a triggering signal for re-routing is received during a lung-related navigation procedure that comprises a pre-procedurally planned pathway to a target location: As a first sub-step 208 of a second step, a current location of the interventional device in relation to the pre-operative 3D model is provided. As a second sub-step 210 of the second step, a plurality of pathway candidates is calculated from the current location to the target location taking into account the at least one 3D model and the associated physical parameters of the at least one available interventional device. For the pathway candidates' calculation, it is provided a step 212 of simulating a plurality of pathway candidates. The simulating comprises as a first sub-step 214 the step of determining device-pathway compatibility of the simulated pathway candidates; and as a second sub-step 214 the step 216 of evaluating the simulated pathway candidates for selection of a best candidate.

In an example of the method, for the simulating, at least one parameter of the group of: type of the interventional device, flexibility of the interventional device, radius of the interventional device, imaging device type, device tip size, needle or forceps size and navigational pathway, is varied.

In an example of the method, for the evaluating, at least one of the following criteria is measured:
- amount of target tissue able to be biopsied;
- avoidance of critical surrounding comprising at least one of the group of critical vasculature and critical nerve tracts;
- pathway navigability; and
- fluoroscopy view quality.

In an example of the method, possible simulated pathway candidates are provided to the user for user selection.

In an example of the method, the measured criteria are numerically assessed and wherein the simulated pathway with the highest scoring is selected and suggested as a re-routed pathway for updated navigation to the target location.

In an example of the method, for the providing of the pre-operative 3D model of at least a part of the tree structure, it is provided the steps of:
- providing a pre-operative 3D model of at least a part of a lung of a subject currently undergoing the lung-related procedure; and
- segmenting the airways from the pre-operative 3D model; and
- extracting the tree structure of the airways.

In an example of the method, a set of available devices in the operating room is provided. A plurality of 3D models for the available interventional devices is provided. For the simulating, the variation of at least one parameter of the group of: type of the interventional device, flexibility of the interventional device, radius of the interventional device, imaging device type, device tip size, needle or forceps size and navigational pathway, is based upon the set of the available devices.

In an example of the method, a change-indicator is provided to the user if a simulated pathway is determined that is based upon one of the available, but not currently used devices. The change-indicator informs the user that a device change is required for continuing in navigating the device towards the target.

In an example of the method, a simulated fluoroscopy view of the target site for a given approach angle of the respective simulated pathway is generated and presented to the user.

In an example of the method, for the evaluating, a user's prior experience and expertise is taken into account for recommending a pathway and device combination.

In an example, a computer program comprising instructions is provided, which, when the program is executed by a computer, cause the computer to carry out the method of one of the preceding examples.

In an example, a computer program or program element for controlling an apparatus according to one of the examples above is provided, which program or program element, when being executed by a processing unit, is adapted to perform the method steps of one of the method examples above.

In an example, a computer readable medium having stored the computer program of the preceding example is provided.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for re-routing a planned pathway for an interventional device in lung-related interventions, the device comprising:
- a data input (12);
- a data storage (14);
- a data processor (16); and
- an output interface (18);
wherein the data input is configured to provide a current location of the interventional device in relation to the pre-operative 3D model;
wherein the data storage is configured: to provide a pre-operative 3D model of at least a part of the airways of a subject currently undergoing a lung-related procedure; and to provide at least one 3D model of at least one available interventional device and physical parameters associated with the at least one available interventional device;
wherein the data processor is configured: to calculate a plurality of pathway candidates from the current location to the target location taking into account the at least one 3D model and the associated physical parameters of the at least one available interventional device upon receiving a triggering signal for re-routing during a lung-related navigation procedure that comprises a pre-procedurally planned pathway to a target location; wherein, for the pathway candidates' calculation, the data processor is configured to simulate a plurality of pathway candidates; and wherein, for the simulating, the data processor is configured to determine device-pathway compatibility of the simulated pathway candidates; and to evaluate the simulated pathway candidates for selection of at least one candidate; and
wherein the output interface is configured to provide at least one simulated pathway candidate for further navigation of the interventional device.

2. Device according to claim 1, wherein the physical parameters comprise at least one of the group of: flexibility of the device, variations of the flexibility along the length of the device, bending radius of the device, variations of the bending radius along the length of the device, surface smoothness and resulting friction with adjacent or abutting tissue surfaces and size of the device.

3. Device according to claim 1 or 2, wherein, for the simulating, at least one parameter of the group of: type of the interventional device, flexibility of the interventional device, radius of the interventional device, imaging device type, device tip size, needle or forceps size and navigational pathway, is varied.

4. Device according to claim 1, 2 or 3, wherein the data input is configured to receive a user interaction for identifying a location of a blockage in the airways of the subject; and
wherein the data processor is configured to consider the blockage when simulating the pathway candidates.

5. Device according to one of the preceding claims, wherein possible simulated pathway candidates are provided to the user for user selection.

6. Device according to one of the preceding claims, wherein, for the evaluating, at least one of the following criteria is measured: amount of target tissue able to be biopsied, avoidance of critical surroundings comprising at least one of the group of critical vasculature and critical nerve tracts, pathway navigability and fluoroscopy view quality.

7. Device according to one of the preceding claims, wherein the data processor is configured: to numerically assess the measured criteria; to select the simulated pathway with the highest scoring; and to suggest the pathway as a re-routed pathway for an updated navigation to the target location.

8. Device according to one of the preceding claims, wherein a plurality of 3D models of a set of available devices in the operating room is provided; and
wherein, for the simulating, the data processor is configured to base the variation of at least one parameter upon the set of the available devices.

9. Device according to one of the preceding claims, wherein the data processor is configured to provide a change-indicator to the user if a simulated pathway is determined that is based upon one of the available, but not currently used devices; and
wherein the change-indicator informs the user that a device change is required for continuing in navigating the device towards the target along the simulated pathway.

10. Device according to one of the preceding claims, wherein the data processor is configured to generate a simulated fluoroscopy view of the target site for a given approach angle of the respective simulated pathway and to present same to the user.

11. Device according to one of the preceding claims, wherein, for the evaluating, the data processor is configured to take a user's prior experience and expertise into account for recommending a pathway and device combination.

12. A system (50) for lung-related navigation guidance, comprising:
- a device (10) for re-routing a planned pathway for an interventional device in lung-related interventions according to one of the preceding claims;
- an interventional device position determining arrangement (52);
- a user interaction interface (54); and
- a display arrangement (56);
wherein the user interaction interface is configured for generating a triggering signal for re-routing during a lung-related navigation procedure;
wherein the interventional device position determining arrangement is configured to determine a current location of the interventional device upon the triggering signal is generated;
wherein the device for re-routing a planned pathway provides at least one simulated pathway acting as navigation for pursuing with moving the interventional device towards the target; and wherein the display arrangement is configured to provide the at least one simulated pathway.

13. System according to claim 12, wherein an interventional device (72) is provided for moving along inside airway structures towards a target.

14. A method (200) for re-routing a planned pathway for an interventional device in lung-related interventions, the method comprising the following steps:
- providing (202) a pre-operative 3D model of at least a part of the airways of a subject currently undergoing the lung-related procedure; and providing (204) at least one 3D model of at least one available interventional device and physical parameters associated with the at least one available interventional device;
upon receiving (206) a triggering signal for re-routing during a lung-related navigation procedure that comprises a pre-procedurally planned pathway to a target location:
- providing (208) a current location of the interventional device in relation to the pre-operative 3D model;
- calculating (210) a plurality of pathway candidates from the current location to the target location taking into account the at least one 3D model and the associated physical parameters of the at least one available interventional device;
wherein, for the pathway candidates' calculation, it is provided simulating (212) a plurality of pathway candidates; and
wherein the simulating comprises the steps of:
- determining (214) device-pathway compatibility of the simulated pathway candidates; and evaluating (216) the simulated pathway candidates for selection of a best candidate.

15. Computer program comprising instructions which, when the program is executed by a computer, cause the computer to carry out the method of claim 14.
